# EUROPÄISCHE PATENTSCHRIFT

(11) **EP 3 371 319 B1**
(45) Veröffentlichungstag und Bekanntmachung des Hinweises auf die Patenterteilung: **17.07.2019**
(21) Anmeldenummer: 16801701.0
(22) Anmeldetag: 04.11.2016
(51) Int. Cl.: C12P 21/02, C12M 1/00, A23C 9/12, A61K 9/28

(54) **VERFAHREN ZUR HERSTELLUNG TROCKENER DARREICHUNGSFORMEN EINER GCMAF-ENTHALTENDEN FORMULIERUNG**
METHOD FOR PRODUCING DRY DOSAGE FORMS OF A GCMAF-CONTAINING FORMULATION
PROCÉDÉ POUR LA PRODUCTION DE FORMES GALÉNIQUES SÈCHES D'UNE FORMULATION CONTENANT GCMAF

(30) Priorität: 05.11.2015 DE 102015014575
(43) Veröffentlichungstag der Anmeldung: 12.09.2018
(73) Patentinhaber: Granolis GmbH, 99423 Weimar (DE)
(72) Erfinder: KRAH, Henry, 98673 Merbelsrod (DE); JACOB, Michael, 99425 Weimar (DE); BÖBER, Reinhard, 99425 Weimar (DE)
(74) Vertreter: Patentanwälte Magenbauer & Kollegen Partnerschaft mbB
(86) Internationale Anmeldenummer: PCT/DE2016/100521
(87) Internationale Veröffentlichungsnummer: WO 2017/076396

(56) Entgegenhaltungen:
- EP-A1- 2 735 616
- ANONYM.: "Active B®AVO probiotic Easy Kit, Instructions for the preparation of BRAVO yogurt", , 31. März 2015 (2015-03-31), XP002767738, Gefunden im Internet: URL:https://www.drreinwald.de/fileadmin/me dia/downloads/Preparation_Active_Bravo_pro biotic_EASY_KIT-EN.pdf [gefunden am 2017-03-01]
- ANONYM.: "BRAVO Probiotic Yoghurt", , 27. Februar 2015 (2015-02-27), XP002767739, Gefunden im Internet: URL:http://web.archive.org/web/20150227113 422/https://tagc.com.au/bravo-probiotic/ [gefunden am 2017-02-28]
- DATABASE WPI Week 201102 Thomson Scientific, London, GB; AN 2010-P25539 XP002767740, & KR 2010 0118164 A (UNIV WOOSONG CORP IND EDUCATIONAL PROGRA) 5. November 2010 (2010-11-05)
- BETZ, J.M. ET AL.: "The NIH analytical methods and reference materials program for dietary supplements", ANALYTICAL AND BIOANALYTICAL CHEMISTRY, Bd. 389, Nr. 1, 31. Mai 2007 (2007-05-31), Seiten 19-25, XP019537526, DOI: 10.1007/S00216-007-1342-8
- DATABASE EMBASE [Online] ELSEVIER SCIENCE PUBLISHERS; Oktober 2014 (2014-10), THYER, L. ET AL.: "Clinical experience of immunotherapy based on oleic acid bound to glycosylated vitamin D-binding protein in localised and metastatic adenocarcinoma of the pancreas", XP002767741, Database accession no. 0051779787
- ANONYM.: "Bravo Probiotic Concentrate Capsules 30 day supply 46 Probiotics", , 2016, XP002767742, Gefunden im Internet: URL:https://www.bravo-probiotic-yogurt.com /product/bravo-probiotic-concentrate-capsu les [gefunden am 2017-03-01]

## Beschreibung

### Stand der Technik

Die Erfindung geht aus von einem Verfahren zur Herstellung trockener Darreichungsformen einer GcMAF-enthaltenden Formulierung mit den kennzeichnenden Merkmalen des Patentanspruchs 1.

GcMAF, auch DBP-MAF genannt, ist ein Makrophagen-Aktivierender Faktor (MAF), der im menschlichen Körper enzymatisch aus DBP, kurz für Vitamin D-Binde-Protein, hergestellt wird. Hierbei ist DBP ein Glykoprotein mit einem Molekulargewicht von ungefähr 58.000 Dalton und gehört zur Gruppe der Albumine, die im Blut in größerer Menge vorkommen, wobei das DBP Vitamin-D-Derivate bindet und ihren Transport im Blutstrom vermittelt. Die DBP-Blutkonzentration liegt etwa bei 300 - 600 *µ*g/ml. Die Zucker-Seitenkette besteht aus N-Acetylgalactosamin, das mit Galactose und Sialinsäure vernetzt ist. Nach Immunaktivierung wird die Zuckerkette des GC-Proteins durch eine induzierbare Membran-ß-Galactosidase von B-Zellen hydrolysiert und der Makrophagen-Proaktivator entsteht. Das Pro-Aktivatorprotein wird dann durch eine membranständige Sialidase (Neuraminidase) aktivierter T-Zellen zum endgültigen GcMAF mit N-Acetylgalactosamin (GalNAc) als verbleibendem Zuckerrest hydrolysiert. Der GalNAc-Rest ist essentiell für die Wirksamkeit des GcMAF.

Das Enzym "Nagalase" (alpha-N-Acetylgalactosaminidase) ist ein lysosomales Enzym aus der Leber, das die Verbindung zwischen GalNAc und dem Threonin- oder Serinrest von Proteinen spaltet. Die Nagalase kann zudem die Zuckerseitenkette des Gc-Proteins abspalten, wodurch dieses seine Präkursoraktivität verliert und nicht mehr zum aktiven GcMAF umgewandelt werden kann. Darüber hinaus inaktiviert Nagalase auch aktives GcMAF durch Zuckerabspaltung.

Makrophagen besetzen eine Schlüsselrolle in der Immunabwehr und gelangen als junge, unreife Monozyten aus dem Knochenmark ins Blut. Im Blut differenzieren sie sich teilweise zu dendritischen Zellen oder wandern nach Aktivierung in Gewebe aus, wo sie zu residenten Gewebsmakrophagen ausreifen, die dann je nach Gewebe als Kupffer'sche Sternzellen (Leber), Gliazellen (Nervensystem), Alveolarmakrophagen (Lunge), Osteoklasten (Knochen) oder auch als Langhans-Riesenzellen (Haut) bezeichnet werden.

GcMAF hat offensichtlich ein enormes Aktivierungspotential für Monozyten und Makrophagen. Schon geringste Konzentrationen stimulieren die antimikrobielle Produktion von Sauerstoffradikalen, den "oxidativen Burst" von Monozyten bis zu 50-fach. Die Phagozytoseaktivität und die Expression von HLA-Molekülen wird bis zu 100-fach erhöht, wodurch die Präsentation von Antigenen exponentiell gesteigert werden kann. Durch diesen Booster-Effekt können Erreger, die der Immunabwehr zuvor möglicherweise nur eingeschränkt zugänglich waren, effizienter bekämpft werden. Die Immunantwort gegenüber Antigenen, die nicht erkennbar waren, kann aktiviert werden. Tumorzellen, die sich der Immunerkennung entzogen haben können demaskiert und abgetötet werden. Mehrfach konnte in den letzten Jahren bestätigt werden, dass GcMAF die Behandlung von Tumorerkrankungen und von Infektionen wie sogar HIV enorm verbesserte.

Gc-MAF fällt unter das Arzneimittelgesetz, da es intravenös angewandt wird. Da das Präparat noch keine Zulassung als Arzneimittel hat, ist eine Applikation am Patienten nur möglich im Rahmen der Eigenherstellung durch den behandelnden Arzt nach § 13 AMG (Gesetz über den Verkehr mit Arzneimitteln). Voraussetzungen hierbei sind:
▪ Nachweis der Berechtigung zur Ausübung des Heilberufes durch Vorlage der Approbation und der Arztnummer,
▪ Überwachung der Herstellung in einem Kontraktlabor durch den Arzt,
▪ analytische Untersuchungen in einem Fremdlabor, welche die absolute Reinheit und Ungefährlichkeit des Proteins beweisen, und
▪ Nachweis der Aktivität.

GcMAF kann nach der Eigenherstellung durch den behandelnden Arzt momentan in den nachstehend aufgeführten drei Formen
▪ intravenös,
▪ in joghurtartiger Form (orale Verabreichungsform bzw. als Zäpfchen) oder
▪ in gefriergetrockneter Form (sublingual)
verabreicht werden.

Im Dokument "Active BRAVO probiotic Easy Kit, Instructions for the preparation of BRAVO yogurt" vom 31. März 2015 wird ein Verfahren zur Herstellung von Darreichungsformen einer GcMAF-enthaltenden Formulierung und eine für das Verfahren geeignete Vorrichtung offenbart.

Auch im Dokument "BRAVO Probiotic Yoghurt" vom 27. Februar 2015 wird ein Verfahren zur Herstellung von Darreichungsformen einer GcMAF-enthaltenden Formulierung und eine für das Verfahren geeignete Vorrichtung offenbart.

Beide Dokumente offenbaren ein Verfahren zur Herstellung von Darreichungsformen einer GcMAF-enthaltenden Formulierung, dadurch gekennzeichnet,
- dass Starterkulturen in Milch gegeben werden, so dass ein Fermentationsansatz entsteht, wobei anschließend eine Fermentation in der Milch erfolgt.

Die Offenlegungsschrift KR 2010 0118164 A zeigt ein Verfahren und eine Vorrichtung zur Herstellung trockener Darreichungsformen einer fermentierten Milch-enthaltenden Formulierung, wobei das Fermentationsprodukt konvektiv getrocknet wird.

Auch an diesen technischen Lösungen ist nachteilig, dass das Verfahren und die Vorrichtung weder geeignet sind die Lagerstabilität des Produkts zu erhöhen noch eine großtechnische Herstellung zu ermöglichen.

Der Erfindung liegt daher die Aufgabe zugrunde ein verbessertes Verfahren zur Herstellung von Darreichungsformen einer GcMAF-enthaltenden Formulierung, bei denen Starterkulturen in Milch gegeben werden, sodass ein Fermentationsansatz entsteht, wobei anschließend eine Fermentation in der Milch erfolgt, bereitzustellen.

Die Aufgabe wird durch ein Verfahren mit den Merkmalen des Patentanspruchs 1 gelöst.

### Die Erfindung und ihre Vorteile

Das erfindungsgemäße Verfahren zur Herstellung trockener Darreichungsformen einer GcMAF-enthaltenden Formulierung mit den kennzeichnenden Merkmalen des Patentanspruchs 1 hat demgegenüber den Vorteil, dass das Verfahren eine großtechnische Herstellung einer in seiner Aufnahmeform für den Menschen verbesserten Darreichungsform, z. B. als ggf. beschichtete Tablette oder dergleichen, gestattet und somit auch eine kostengünstigere Herstellung des Produkts. Ein weiterer Vorteil ist, dass die getrocknete Darreichungsform biologisch hochaktiv immunmodulatorische Wirkung ausübt und somit die Einnahmehäufigkeit gesenkt werden kann.

Weitere Vorteile der trockenen Darreichungsformen sind unter anderen bspw. eine längere Lagerstabilität, eine geringere Oxidationsempfindlichkeit, eine definierte und einstellbare Partikelstruktur durch die Trocknung selbst bzw. durch die Weiterverarbeitung des getrockneten Materials als
▪ Pulver (beschichtet, unbeschichtet, magensaftresistent, geschmacksmaskiert oder dergleichen),
▪ Granulat (beschichtet, unbeschichtet, magensaftresistent, geschmacksmaskiert oder dergleichen),
▪ Pellet (beschichtet, unbeschichtet, magensaftresistent, geschmacksmaskiert oder dergleichen),
▪ Extrudat (beschichtet, unbeschichtet, magensaftresistent, geschmacksmaskiert oder dergleichen),
▪ Zäpfchen und
▪ Feinstpulver nach Mikronisierung (z. B. durch Mahlung).

Zusätzlich ist der Wirkmechanismus und der Wirkungsort über die Formulierung bzw. die Darreichungsform frei einstellbar. Die Dosierung ist leicht einstellbar über die Formulierung der Darreichungsform, wodurch eine sichere Applikation erzielt wird und Fehler verhindert werden können. Darüber hinaus besteht die Möglichkeit einer leichten Weiterverarbeitbarkeit in unterschiedliche Darreichungsformen, wie beispielsweise
▪ Tablette (beschichtet z. B. magensaftresistent oder als ODT-Tablette),
▪ Kapsel (magensaftresistentes Kapselmaterial oder beschichtete Kapsel),
▪ Zäpfchen und
▪ Pulver.

Des Weiteren kann die feste Darreichungsform im Handel als Nahrungsergänzungsmittel, Neutrazeutical vertrieben werden und somit ist eine sichere Handhabung (kein pharmazeutisches Umfeld notwendig) gewährleistet.

Vorteilhaft ist zudem, dass die Aktivstoffe fein dispers (mikronisiert) vorliegen, d. h. im Vergleich zu bekannten Darreichungsformen wird eine signifikante Oberflächenvergrößerung erzielt und damit eine schnellere Aufnahme im Körper z. B. über die Schleimhaut im Mund bei der oralen Verabreichung.

Vorteilhaft ist auch das gleichzeitige Vorhandensein des Proteins (GcMAF) und von Mikroorganismen bzw. Hefen, die aus der Fermentation vorhanden sind. Diese werden parallel zur Trocknung des GcMAF mitimmobilisiert und sind in der Anwendung revitalisierbar. Zusätzlich werden Synergien genutzt, z. B. als Probiotika im Dünndarm (bessere Gesamtflora oder dergleichen) .

Nach einer vorteilhaften Ausgestaltung des erfindungsgemäßen Verfahrens steht der Fermentationsansatz zwei Tage. Vorteilhafterweise wird durch den längeren Zeitraum eine verbesserte Fermentation erzielt, bei der eine Art Joghurt entsteht.

Ein weiterer diesbezüglicher Vorteil wird durch eine zweitägige Lagerung des Fermentationsansatzes bei einer Temperatur von 37 °C erzielt, wodurch eine zusätzliche Verbesserung der Fermentation und somit der Gehalt an GcMAF im nach der Fermentation enthaltenen Zwischenprodukt/Fermentationsprodukt (eine Art Joghurt) weiter erhöht wird.

Das Zwischenprodukt/Fermentationsprodukt wird vor der konvektiven Trocknung aufgeheizt. Durch eine Erwärmung des Zwischenprodukts/Fermentationsprodukts vor der konvektiven Trocknung wird die Fließfähigkeit des zu versprühenden Zwischenprodukts/Fermentationsprodukts verbessert, wodurch das eine homogenere Sprühflüssigkeit erzeugt wird.

Nach einer diesbezüglich vorteilhaften Ausgestaltung des erfindungsgemäßen Verfahrens wird das Zwischenprodukt/Fermentationsprodukt vor der konvektiven Trocknung auf eine Temperatur von 35 °C aufgeheizt. Hierdurch wird eine optimale Fließfähigkeit des Zwischenprodukts/Fermentationsprodukts erzielt, wobei die im Zwischenprodukt/Fermentationsprodukt enthaltenen Proteine ihr Aktivitätsmaximum erreichen, welches meist im Bereich einer Temperatur von 35 bis 39 °C liegt. Die Temperatur entspricht dem Temperaturoptimum, bei dem die Starterkulturen (Organismen) sich am besten vermehren. Ein Überschreiten dieser Temperatur kann zu irreversiblen Schädigungen durch Denaturierung der Proteine führen, eine Unterschreitung führt zu geringeren Stoffwechselgeschwindigkeiten und damit zu längeren Prozessdauern.

Nach einer zusätzlichen vorteilhaften Ausgestaltung des erfindungsgemäßen Verfahrens wird das Zwischenprodukt/Fermentationsprodukt auf Trägermaterial aufgesprüht. Durch das Aufsprühen auf Trägermaterial wird der Prozess der konvektiven Trocknung verbessert, da so auch ein kontinuierliches Anfahren des zur konvektiven Trocknung herangezogenen Apparates gewährleistet werden kann.

Nach einer zusätzlichen vorteilhaften Ausgestaltung des erfindungsgemäßen Verfahrens wird der Fermentationsansatz mit Vitamin D3 angereichert. Das Vitamin D3 setzt sich vorteilhafterweise an eine der drei Bindungsstellen des Makrophagenaktivierungsfaktors (MAF) und aktiviert so das MAF.

Nach einer zusätzlichen vorteilhaften Ausgestaltung des erfindungsgemäßen Verfahrens wird der Fermentationsansatz mit Colostrum angereichert. Colostrum dient hierbei als Starterhilfe für die Fermentierung. Zudem weist das MAF drei Bindungsstellen auf, die durch Vitamin D3, Colostrum und Ölsäure bei der Fermentierung aktiviert werden.

Nach einer zusätzlichen vorteilhaften Ausgestaltung des erfindungsgemäßen Verfahrens wird der Fermentationsansatz mit Ölsäure angereichert. Vorteilhafterweise dient auch die Ölsäure zur Aktivierung des MAF, indem es ebenfalls an eine der drei Bindungsstellen des MAF andockt.

Nach einer zusätzlichen vorteilhaften Ausgestaltung des erfindungsgemäßen Verfahrens erfolgt die Fermentation unter einer Begasung. Vorteilhafterweise werden durch die Begasung des Reaktors (Bioreaktor, Fermenter) die für die Starterkulturen (Organismen) im Nährmedium wichtigen Nährstoffe stets in ausreichender Form zur Verfügung gestellt.

Nach einer zusätzlichen vorteilhaften Ausgestaltung des erfindungsgemäßen Verfahrens erfolgt die Fermentation unter Rühren. Durch Rühren, bspw. durch ein Rührwerk oder eine andere Einrichtung, wird eine homogene Einstellung der Parameter über den gesamten Reaktorraum sichergestellt. Für eine noch vorteilhaftere Ausgestaltung des erfindungsgemäßen Verfahrens erfolgt die Fermentation unter ständigem Rühren.

Nach einer zusätzlichen vorteilhaften Ausgestaltung des erfindungsgemäßen Verfahrens wird die trockene Darreichungsform einer GcMAF-enthaltenden Formulierung beschichtet. Die Beschichtung der trockenen Darreichungsformen einer GcMAF-enthaltenden Formulierung hat den Vorteil, dass bspw. durch die Beschichtung die Möglichkeit besteht den Wirkstoff an einer vorher bestimmten Stelle bzw. zu einer vorher bestimmten Zeit freizusetzen. So kann die trockene Darreichungsformen einer GcMAF-enthaltenden Formulierung bspw. die Magenpassage passieren ohne vorher durch die Magensäfte aufgelöst zu werden. Der Wirkstoff kann dann gezielt im Darm freigesetzt werden und somit optimal vom Körper aufgenommen werden.

Die Vorrichtung zur Herstellung trockener Darreichungsformen einer GcMAF-enthaltenden Formulierung hat den Vorteil, dass die Vorrichtung zur Herstellung trockener Darreichungsformen einer GcMAF-enthaltenden Formulierung eine großtechnische Herstellung einer in seiner Aufnahmeform für den Menschen verbesserten Darreichungsform, z. B. als ggf. beschichtete Tablette oder dergleichen, ermöglicht und somit auch eine kostengünstigere Herstellung der GcMAF-enthaltenden Formulierung gestattet.

Nach einer beispielhaften Ausgestaltung der Vorrichtung ist der mindestens eine Reaktor für den Fermentationsprozess begasbar. Durch die Begasung des Reaktors werden vorteilhafterweise den Starterkulturen (Organismen) während des Fermentationsprozesses die benötigten Nährstoffe stetig zur Verfügung gestellt, so dass ein optimaler Fermentationsprozess stattfinden kann.

Nach einer zusätzlichen beispielhaften Ausgestaltung der Vorrichtung weist der mindestens eine Reaktor (Bioreaktor/Fermenter) für den Fermentationsprozess eine Rühreinrichtung, bspw. ein Rührwerk oder eine andere geeignete Vorrichtung zum Rühren, auf. Durch eine Rühreinrichtung wird eine homogene Einstellung der Betriebsparameter, die in dem natürlichen Lebensraum der Organismen herrschen, wie die Art und Konzentration der Nährstoffe, die Temperatur, der Sauerstoffgehalt der pH-Wert etc., über den gesamten Reaktorraum sichergestellt. Somit wird durch den Einsatz einer Rühreinrichtung das Fermentationsprodukt deutlich in seiner Qualität verbessert.

Nach einer diesbezüglich beispielhaften Ausgestaltung der Vorrichtung ist der mindestens eine Reaktor für den Fermentationsprozess temperierbar. Die Starterkulturen (Organismen) haben ein Temperaturoptimum, bei dem sie sich am besten vermehren. Ein Überschreiten dieser Temperatur kann zu irreversiblen Schädigungen durch Denaturierung der Proteine führen, eine Unterschreitung führt zu geringeren Stoffwechselgeschwindigkeiten und damit zu längeren Prozessdauern. Die Temperaturregelung wird durch Heiz- und Kühlkreisläufe realisiert. Beim Anfahren des Reaktors wird der gesamte Reaktorinhalt auf Betriebstemperatur geheizt bzw. gewärmt. Teilweise erzeugen die kultivierten Organismen durch ihren Stoffwechsel so viel Abwärme, dass ab einer bestimmten Zellkonzentration nur noch der Kühlkreislauf aktiv ist. In diesen Kreislauf kann ein Wärmetauscher integriert sein oder das energietragende Medium wird direkt eingespeist. Als Wärmeaustauschflächen zum Reaktionsraum stehen hierbei meist nur die doppelte Behälterwand, in seltenen Fällen auch eingebaute Kühlregister, zur Verfügung. Durch die Möglichkeit der Temperierung des Reaktors, bspw. nach einer einem Fachmann bekannten Art und Weise, wird somit ebenfalls ein hochwertiges Fermentationsprodukt gewährleistet, wobei die Betriebsbedingungen optimal auf den jeweiligen Prozessverlauf einstellbar sind.

Nach einer diesbezüglich beispielhaften Ausgestaltung der Vorrichtung ist die mindestens eine konvektive Trocknungseinheit ein Fluidisierungsapparat, insbesondere in Form einer Wirbelschicht, einer Strahlschicht, einer Sprühtrocknung, insbesondere der Sprühgranulation.

Weitere Vorteile und vorteilhafte Ausgestaltung der Erfindung sind der nachfolgenden Beschreibung, den Ansprüchen und den Zeichnungen entnehmbar.

### Zeichnung

Bevorzugte Ausführungsbeispiele des erfindungsgemäßen Gegenstands sind in der Zeichnung dargestellt und werden im Folgenden näher erläutert. Es zeigen
- Fig. 1a: Prozessparameter der Versuche 001 und 002,
- Fig. 1b: weitere Prozessparameter der Versuche 001 und 002,
- Fig. 1c: weitere Prozessparameter der Versuche 001 und 002,
- Fig. 2a: Prozessparameter der Versuche 003 und 004,
- Fig. 2b: weitere Prozessparameter der Versuche 003 und 004,
- Fig. 2c: weitere Prozessparameter der Versuche 003 und 004,

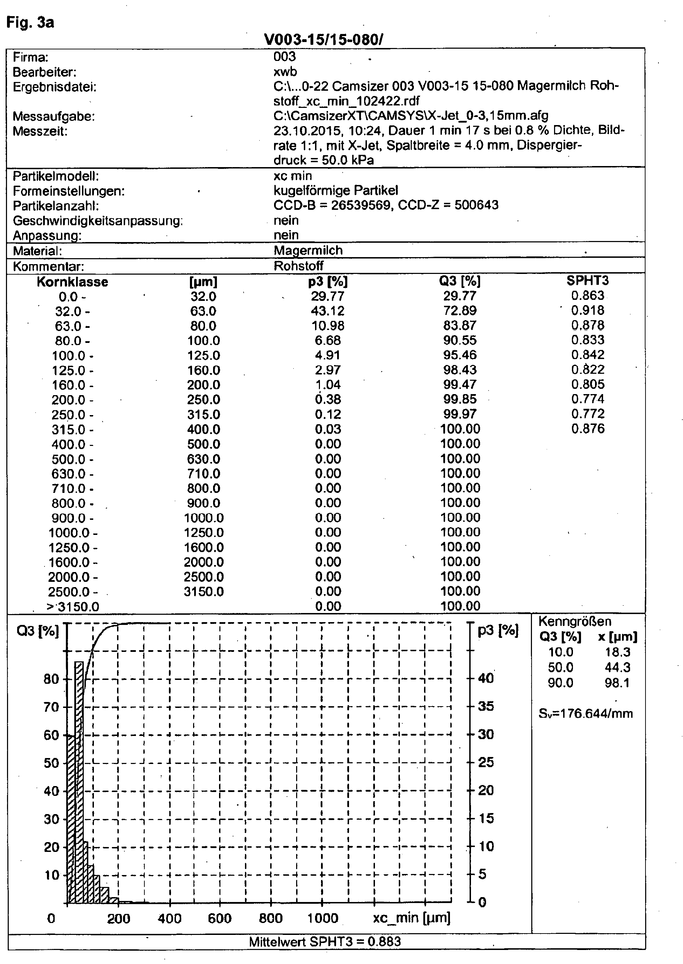

- Fig. 3a: ein Analysenergebnis eines Versuchs,

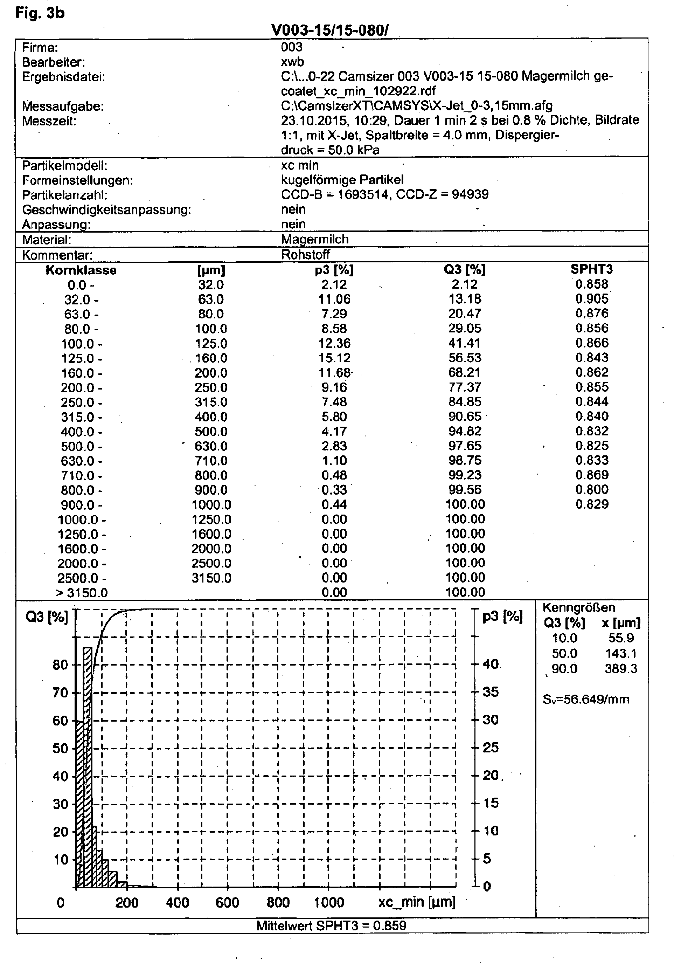

- Fig. 3b: ein Analysenergebnis eines zweiten Versuchs,

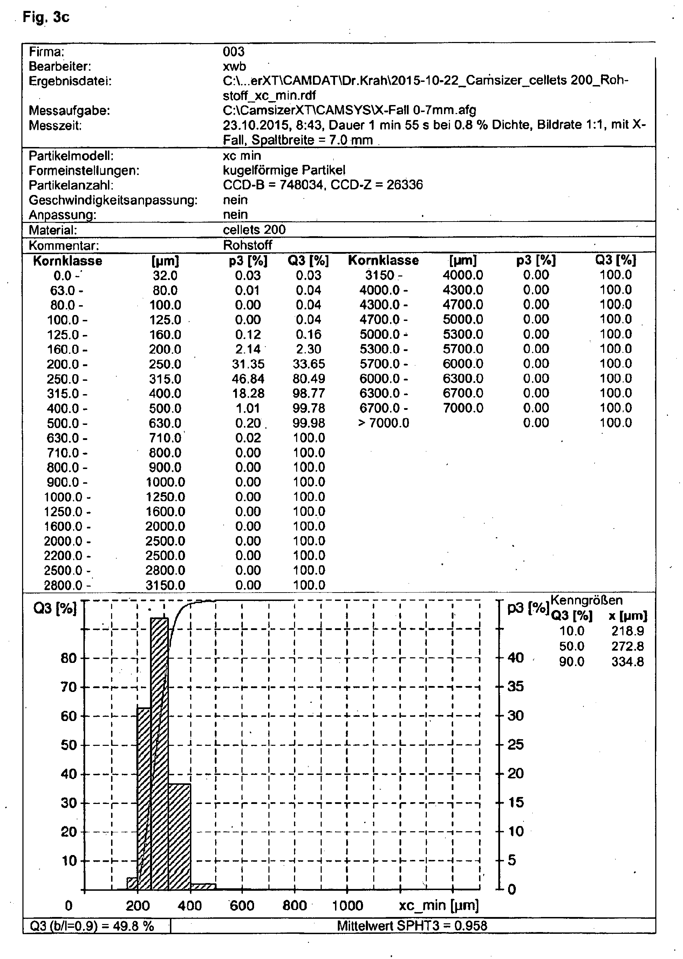

- Fig. 3c: ein Analysenergebnis eines dritten Versuchs,

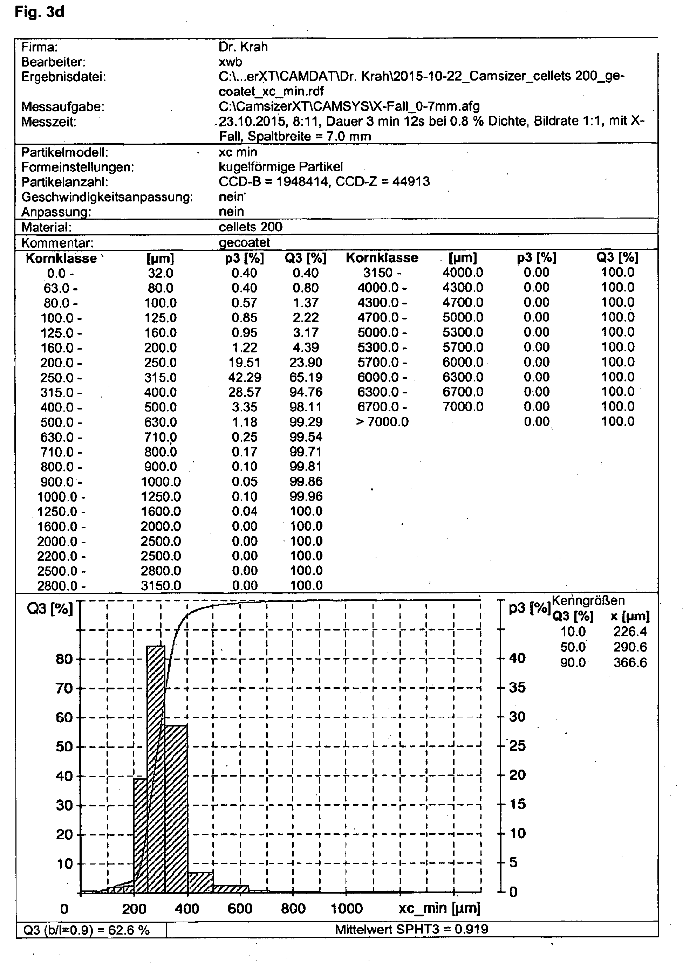

- Fig. 3d: ein Analysenergebnis eines vierten Versuchs,

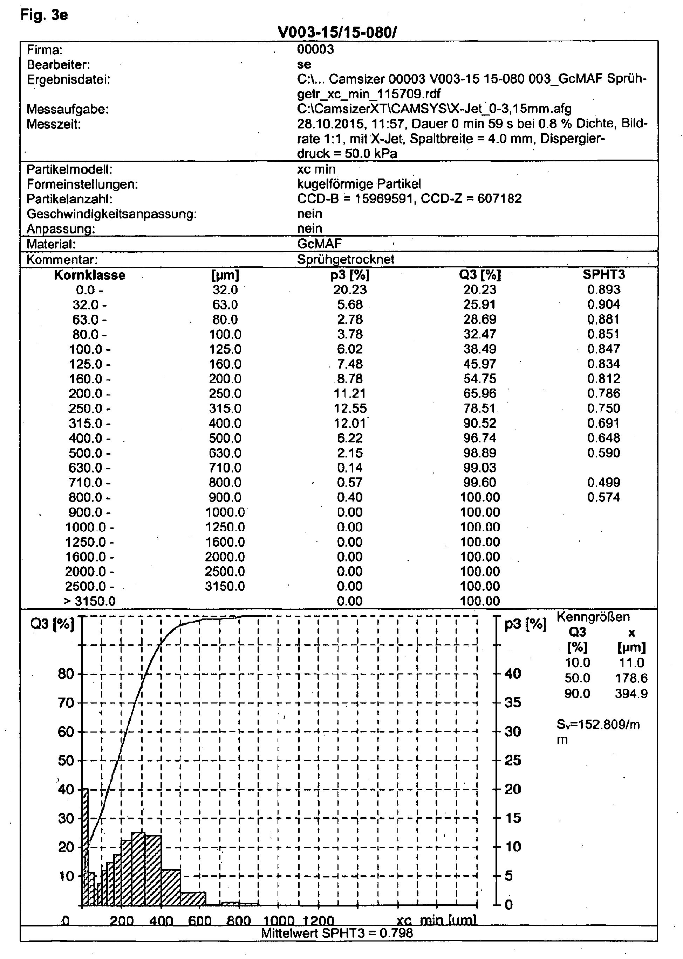

- Fig. 3e: ein Analysenergebnis eines fünften Versuchs,

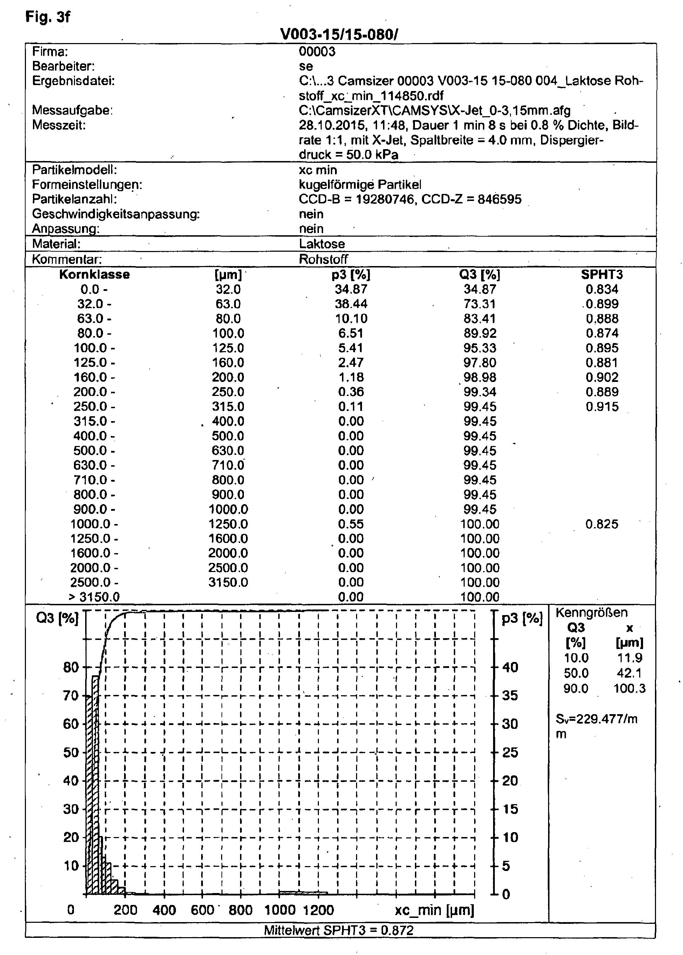

- Fig. 3f: ein Analysenergebnis eines sechsten Versuchs,

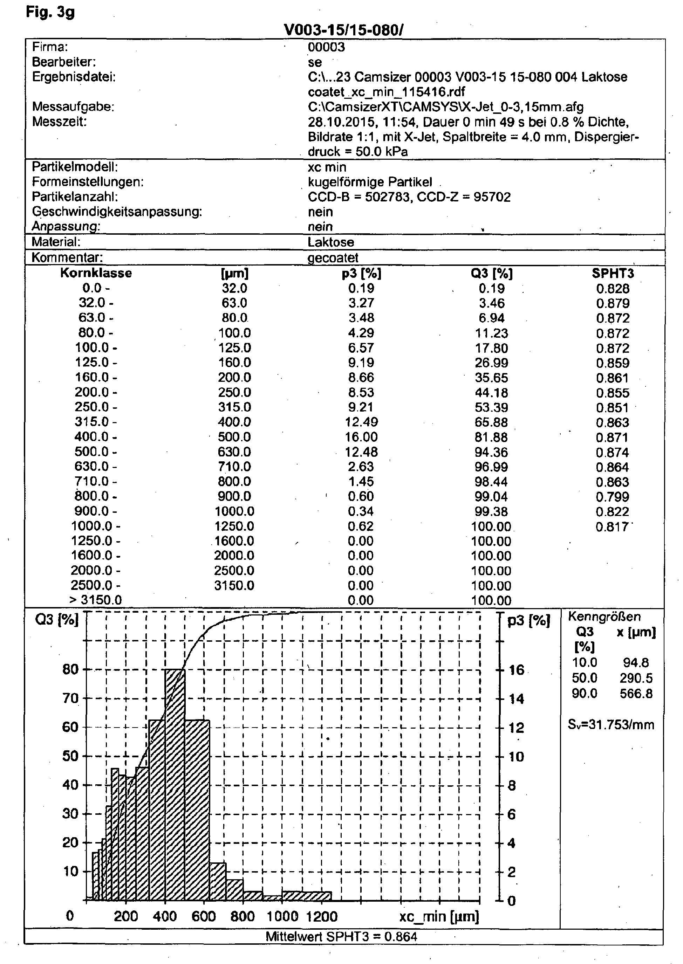

- Fig. 3g: ein Analysenergebnis eines siebten Versuchs,
- Fig. 4: zwei Mikroskopaufnahmen von Magermilchpulver (V003-15/15-080/001),
- Fig. 5: zwei Mikroskopaufnahmen von Cellets 200 (V003-15/15-080/002),
- Fig. 6: eine Mikroskopaufnahme von GcMAF-sprühgetrocknet (V003-15/15-080/003),
- Fig. 7: zwei Mikroskopaufnahmen von Laktose (V003-15/15-080/004),
- Fig. 8: einen ersten ärztlichen Befundbericht,
- Fig. 9: einen zweiten ärztlichen Befundbericht und
- Fig. 10: einen dritten ärztlichen Befundbericht.

### Beschreibung der Ausführungsbeispiele

Die Vorrichtung und das Verfahren zur Herstellung trockener Darreichungsformen einer GcMAF-enthaltenden Formulierung basieren auf der Fermentation und anschließenden konvektiven Trocknung des durch die Fermentation entstandenen Produkts, wodurch die gewünschte feste Darreichungsform entsteht.

Die GcMAF werden auf Basis von Starterkulturen (Organismen) über Fermentation hergestellt, wobei die Starterkulturen bezogen werden können. Die Fermentation erfolgt in Rohmilch (ähnlich wie die Herstellung von Joghurt). Die Keime werden in Flüssigform für die Fermentation angeliefert. Zusätzlich werden noch Vitamin D3, Colostrum und Ölsäure separat geliefert. Es handelt sich hierbei nicht um infektiöse Keime. Die gelieferte Menge bezieht sich auf 100 Liter Fermentationsansatz. Das gilt auch für Vitamin D3, Colostrum und Ölsäure. Zusätzlich wird Milch, vorzugsweise Bio-Milch, bspw. Milch von Demeter-Bauern benötigt. Die Fermentation erfolgt nicht unter Begasung und auch nicht unter ständigem Rühren. Die Milch wird initial mit den Zusätzen verrührt. Danach steht der Fermentationsansatz 2 Tage bei 37° Celsius. Bei diesem Vorgang entsteht eine Art Joghurt. Eine Probennahme während des Fermentationsprozesses ist nicht notwendig. Das Endprodukt sollte kühl gelagert werden. Hierbei findet die Fermentation in einem Reaktor (Bioreaktor/Fermenter) statt. Der Reaktor kann begast, gerührt und/oder temperiert werden. Nachfolgend aufgeführt ist die Auflistung der Fermentations-Zusammensetzung bezogen auf 1000 ml Milch:
5,7 g Colostrum
Lactobazillen, 6 x 10⁹ KBE (Kolonie bildende Einheiten)
Lactobazillus salivarius
Lactobacillus acidophilus 2x
Lactobacillus paracasei
Lactobacillus rhamnosus
Lactobacillus bulgaricus
Bifidobacterien, 3 x 10⁹ KBE (Kolonie bildende Einheiten)
Bifidobacterium bifidum
Bifidobacterium longum
Bifidobacterium animalis
Bifidobacterium adolescentis
Bifidobacterium breve
Bifidobacterium infantis
Bifidobacterium lactis
Lactobacillus lactis, 2 x 10⁹ KBE (Kolonie bildende Einheiten)
Leuconostoc mesenteroides subsp. cremoris 1 x 10⁹ KBE (Kolonie bildende Einheiten) Hefen
Kluyveromyces 1 x 10⁹
Saccharomyces, 1 x 10⁹
Acetobacter, 1 x 10⁹
Streptococcus thermophilus, 1 x 10⁹ (Starter)

Die Mikronisierung (Trocknung) der Biomasse sollte für leichte Aufnahme, z. B. unter der Zunge (sublingual) dahingehend realisiert werden, dass die Partikelgröße im Mikrometerbereich liegt. Hierzu wurde Probematerial für eigene Wirksamkeitstests hergestellt. Der Ansatz zur Mikronisierung wurde vorgeschlagen, um die Magenpassage zu vermeiden.

Alternativ kann ein Layering der Biomasse auf Starterkern, z. B. Cellet 200 oder dergleichen, und anschließendes magensaftresistentes Coating (z. B. Schellack, Eudragit oder dergleichen) erfolgen. D. h. im Anschluss an die Fermentation wird eine feste Darreichungsform einer GcMAF-enthaltenden Formulierung mittels konvektiver Trocknung (Wirbelschicht, Strahlschicht, Sprühgranulation oder dergleichen) des durch die Fermentation erhaltenen Produkts hergestellt. Die dann entstandene feste Darreichungsform kann beliebig mittels bereits bestehender Verfahren beschichtet werden. Zusätzlich besteht ebenfalls die Möglichkeit die feste Darreichungsform in beliebigen anderen Formen zu verabreichen, bspw. als Kapsel oder dergleichen.

Nachfolgend werden mehrere Ausführungsbeispiele der konvektiven Trocknung und deren Ergebnisse aufgeführt:

### 1 Aufgabenstellung

Das Ziel bestand darin den Trockenstoff aus der gelieferten Sprühlösung durch Sprühgranulationstrocknung zurückzugewinnen.

### 2 Versuchsaufbau

Die Sprühgranulationstrocknung wurde Batchweise auf der Pro-Cell Laboranlage mit dem Vario 3 Einsatz durchgeführt. Über der Schicht waren die Filter angeordnet. Sie wurden kontinuierlich durch Druckluftstöße abgereinigt, so dass der Staub in den Prozessraum zurückbefördert wurde. Die Prozessluft wurde durch den drehzahlgeregelten Abluftventilator gefördert. Für das Aufheizen der Luft wurde ein elektrischer Heizer genutzt. Mit einer Bottomspraydüse (Zweistoffdüse, kalte Düsenluft) wurde die Sprühflüssigkeit verdüst. Die Sprühflüssigkeit wurde vom Vorlagebehälter (gerührt) mit einer Schlauchpumpe zu der Düse befördert.

### 3 Analytische Methoden

Während der Granulationstrocknung wurden Produktproben entnommen um den Prozess besser beurteilen zu können. Die Partikelanalysen wurden mit dem optischem Bildauswerte-System (Camsizer - Retsch) durchgeführt. Zur optischen Beurteilung der Proben wurden zudem Aufnahmen mit dem AXIO Mikroskop von Zeiss gemacht.

### 4 Versuchsdurchführung und Ergebnisse

Die Versuche wurden ohne den Kunden durchgeführt. Die Trendkurven der wichtigsten Prozessparameter im Anhang beschreiben die Versuchsbedingungen. Die Analysen und Mikroskop aufnahmen verdeutlichen die Ergebnisse.

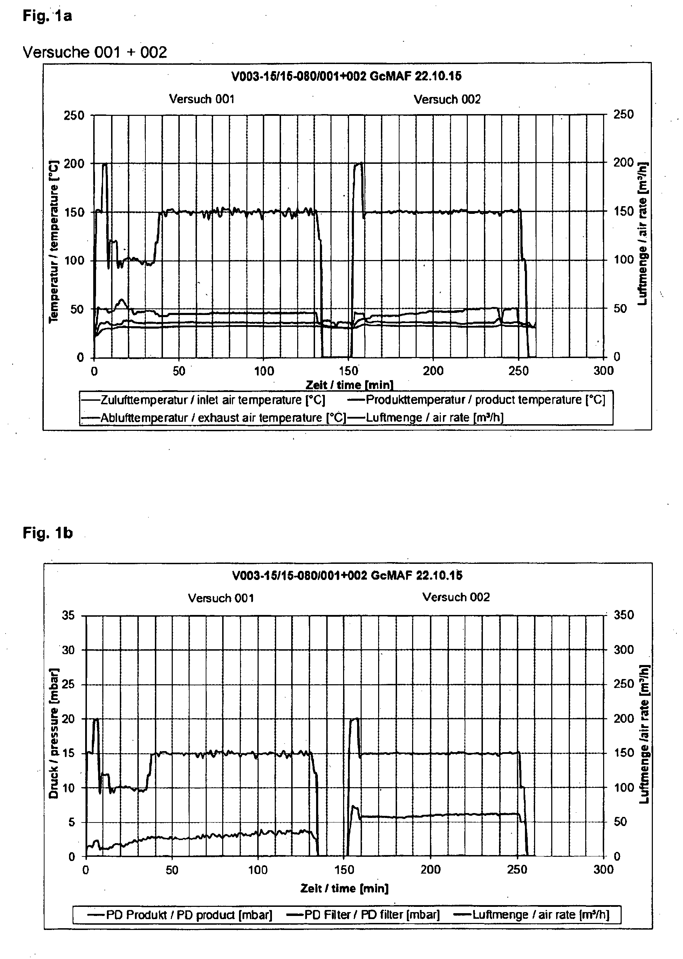

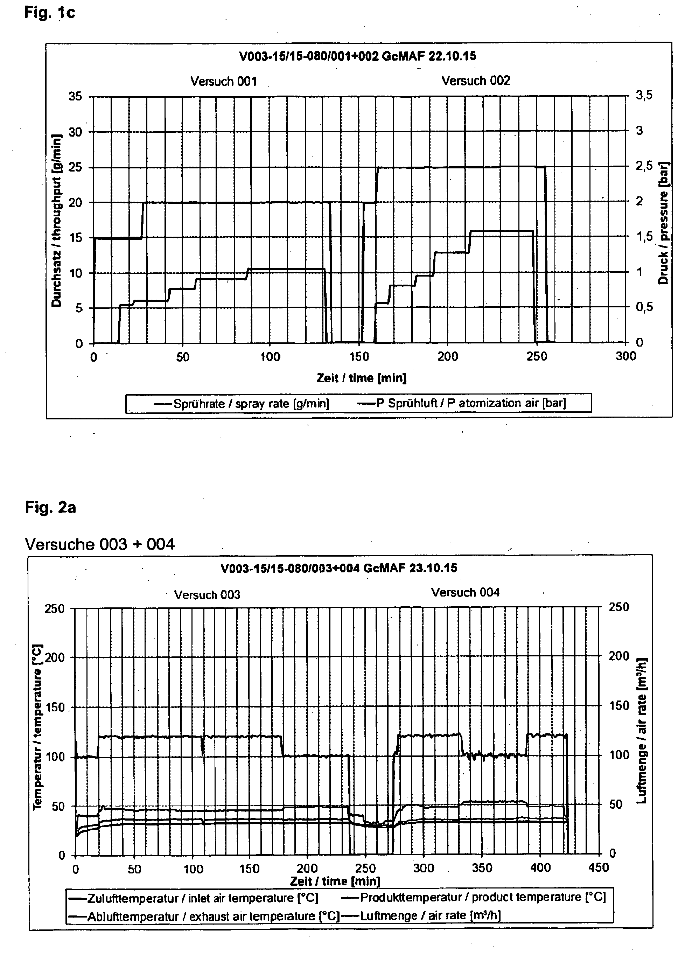

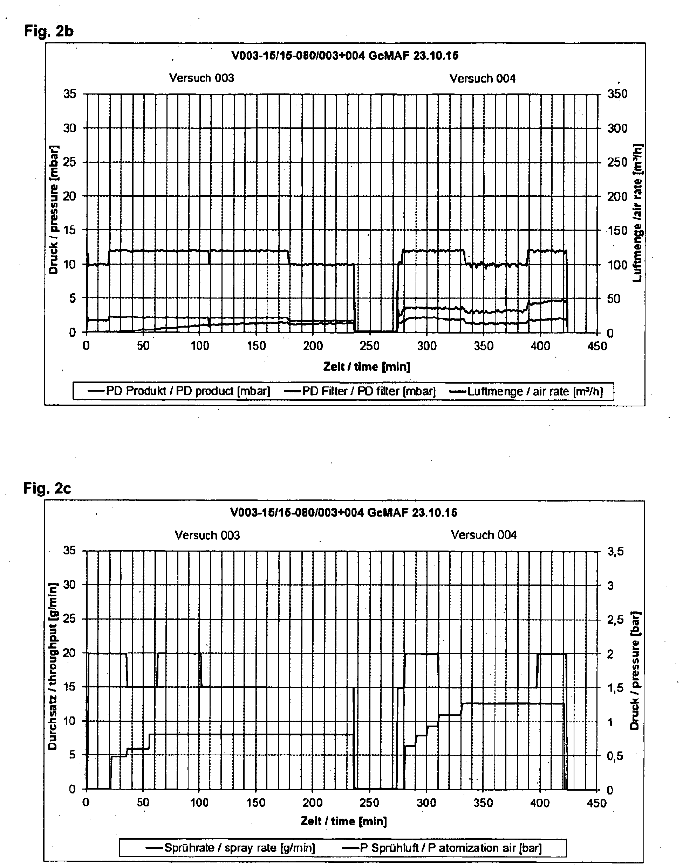

Die Versuche 001 und 002 sind in Fign. 1a bis 1c dargestellt, die Versuche 003 und 004 in Fign. 2a bis 2c.

### V003-15 / 15-080 / 001

Für den ersten Versuch wurden 1000 g Magermilchpulver als Startfüllung vorgelegt. Um die Sprühflüssigkeit versprühen zu können wurde sie mit dem Ultra-Turrax zwei Minuten bei 3000 U/min aufgerührt. 1000 g Sprühflüssigkeit wurden auf das Magermilchpulver aufgebracht. Während des Rührens hatte sich auf der Oberfläche eine gelblich cremige Masse abgesetzt. Der Versuch konnte ohne weitere Probleme durchgeführt werden.

### V003-15 / 15-080 / 002

Beim zweiten Versuch wurden 1000 g Cellets 200 in vorgelegt und das Sprühen gestartet. Auch hier wurden wieder 1000 g Sprühflüssigkeit aufgebracht.

### V003-15 / 15-080 / 003

Im dritten Versuch wurde eine Sprühtrocknung durchgeführt. Neue Produktfilter wurden installiert und das Sprühen ohne Vorlagematerial gestartet. Das hierbei entstanden Trockenprodukt wurde nach Versuchsende von den Behälterwänden und den Filtern abgenommen. Es wurden 1600 g Sprühflüssigkeit versprüht.

### V003-15 / 15-080 / 004

Im vierten Versuch wurden 500 g Laktose vorgelegt. Anders als in den vorherigen Versuchen wurde hier die Sprühflüssigkeit unter ständigem Rühren auf 35° C temperiert. Wie erwartet löste sich die gelblich cremige Masse auf und es entstand eine homogene Sprühlösung. Es wurden 1127 g auf die Laktose aufgesprüht. Bei dem Versuch wurden Partikelgröße mit einem d₅₀ = 178,6 µm erreicht.

### 5 Schlussfolgerungen

Die Sprühgranulationstrocknung ist gut machbar, wenn eine geeignete Startfüllung vorgelegt wird.

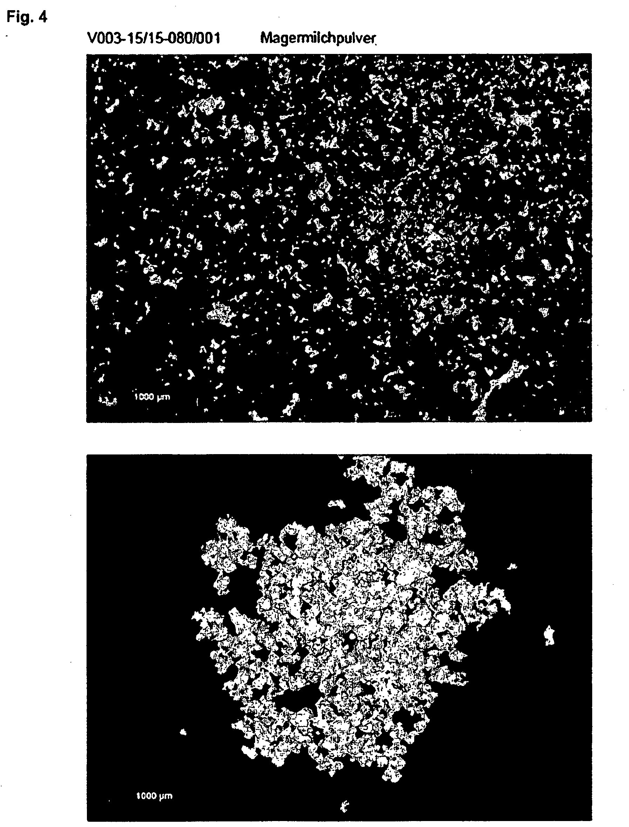

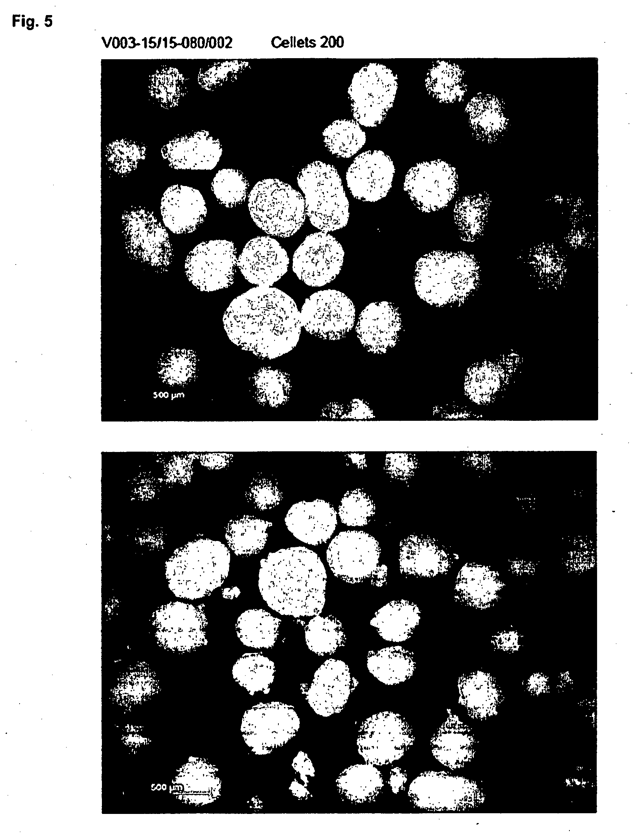

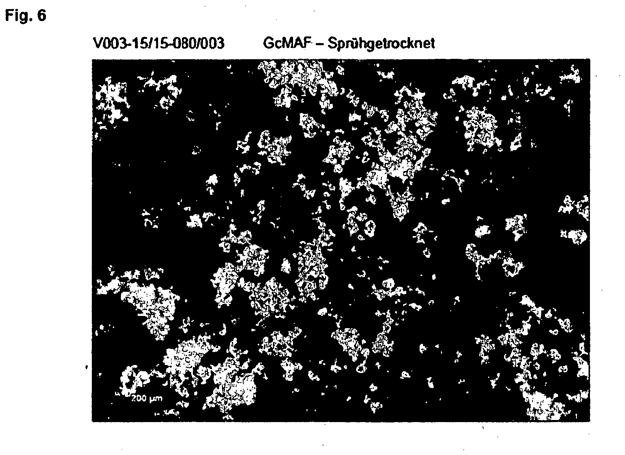

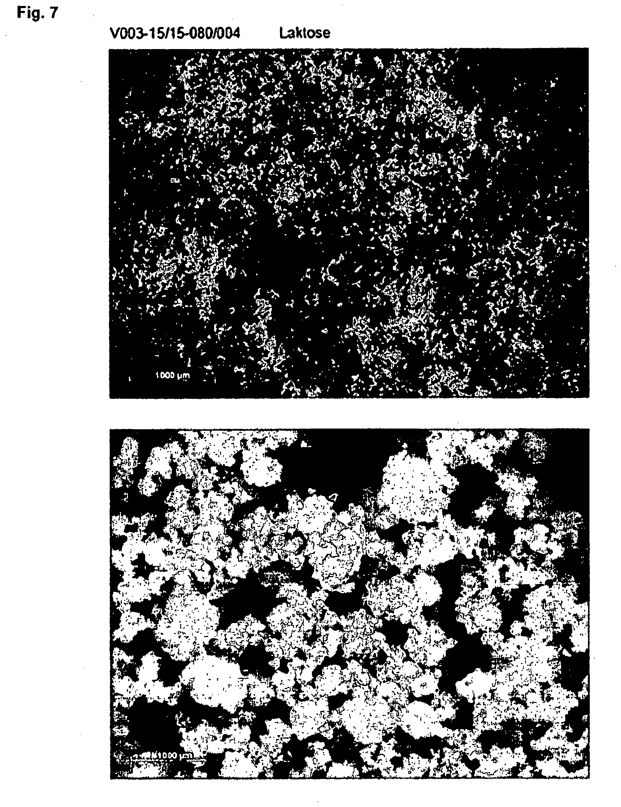

Als Trägermaterial wurden Milchpulver; Laktose und Cellets getestet. Andere Trägermaterialien sind jedoch durchaus denkbar und einsetzbar. Die Figuren 4 bis 7 zeigen das jeweilige beschichte Trägermaterial.

Um ein homogenes Produkt zu erreichen sollte die Sprühflüssigkeit auf 35°C geheizt werden.

Der Start ohne Vorlagematerial wäre zukünftig auch möglich. Es müsste anfangs der Prozess mehrfach unterbrochen werden. Der sich bildende Trockenstoff ist dann von den Behälterwänden abzuschaben, so dass sich eine Wirbelschicht nach und nach bildet. Für einen solchen Versuch ist dann eine Sprühlösungsmenge mit ca. 500 g Trockenstoff zur Verfügung zu stellen.

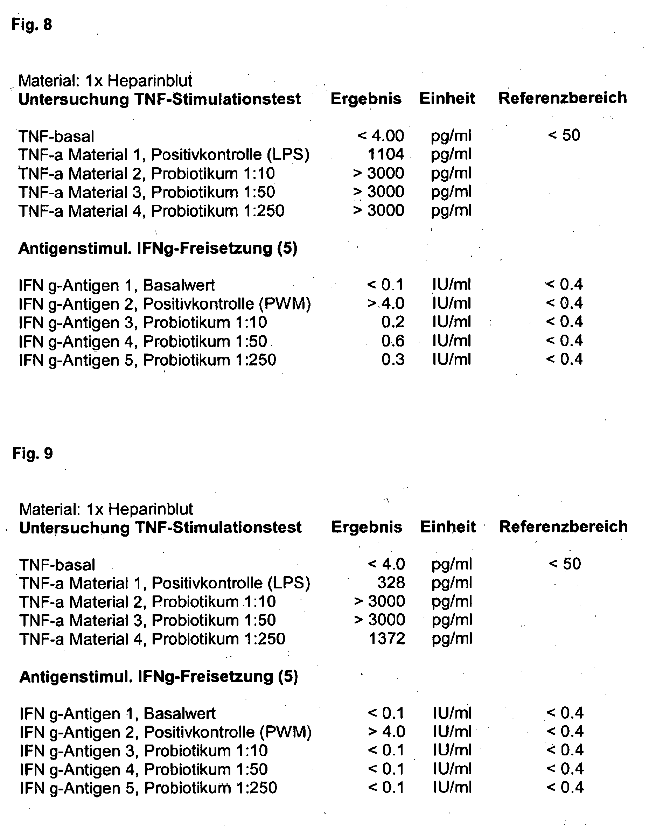

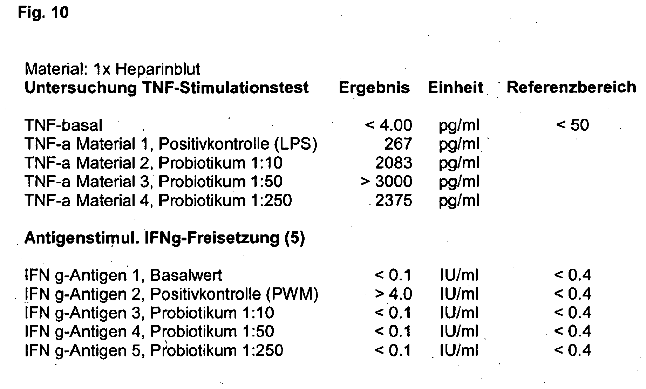

Erläuterung zum Makrophagenstimulationstest mit dem hergestellten Probiotikum in getrockneter Form. Untersucht wurde in einem standardisierten Makrophagenstimulationstest die Wirkung des getrockneten Probiotikums auf die Makrophagenaktivität. Dazu wurden drei unterschiedliche Blutproben getestet, welche in den Fig. 8 bis 10 gezeigt sind. Übereinstimmend zeigte sich in allen Blutproben eine überdeutliche Aktivierung der Makrophagen, gemessen an der TNF-alpha-Titerbestimmung. Somit ist davon auszugehen, dass das Probiotikum in der vorliegenden getrockneten Beschaffenheit biologisch hochaktiv immunmodulatorische Wirkung ausübt.

Der generelle Prozess bzw. der verfahrenstechnische Ansatz besteht aus einer Kombination aus Fermentation von Kulturen (Gemische) und anschließender konvektiver Trocknung zur Herstellung einer stabilisierten bzw. immobilisierten langzeitstabilen festen Form. Das getrocknete Produkt kann unterschiedlich konditioniert werden.

Die verfahrenstechnische Lösung soll einen hygienisch sicheren Prozess abbilden, wobei bspw. alles gekapselt sein kann (GMP-Umgebung leicht möglich). Zudem ist eine leichte inprozess-Kontrolle möglich. Aufgrund des geringen Energieaufwands bei gleichzeitig optimaler Produktsicherheit wird ein kostengünstiges Verfahren zur Herstellung trockener Darreichungsformen einer GcMAF-enthaltenden Formulierung erreicht.

Die thermische konvektive Trocknung kann bspw. durch eine Wirbelschicht, eine Strahlschicht oder eine Sprühtrocknung erreicht werden, wobei alternativ eine Kontakttrocknung denkbar ist. Jedoch ist hierbei keine so definierte Prozessführung möglich. An der Gefriertrocknung nachteilig ist, dass diese eine undefinierte Produktstruktur und hohe Prozesskosten aufweist.

## Patentansprüche

1. Verfahren zur Herstellung trockener Darreichungsformen einer GcMAF-enthaltenden Formulierung, **dadurch gekennzeichnet,**
- **dass** Starterkulturen in Milch gegeben werden, so dass ein Fermentationsansatz entsteht, wobei anschließend eine Fermentation in der Milch erfolgt,
- das Fermentationsprodukt vor der konvektiven Trocknung aufgeheizt wird und
- **dass** danach das Fermentationsprodukt konvektiv getrocknet wird.

2. Verfahren nach Anspruch 1, **dadurch gekennzeichnet, dass** der Fermentationsansatz zwei Tage steht.

3. Verfahren nach Anspruch 2, **dadurch gekennzeichnet, dass** der Fermentationsansatz zwei Tage bei einer Temperatur von 37 °C gelagert wird.

4. Verfahren nach einem der vorhergehenden Ansprüche, **dadurch gekennzeichnet, dass** das Fermentationsprodukt vor der konvektiven Trocknung auf eine Temperatur von 35 °C aufgeheizt wird.

5. Verfahren nach einem der vorhergehenden Ansprüche, **dadurch gekennzeichnet, dass** das Fermentationsprodukt auf Trägermaterial aufgesprüht wird.

6. Verfahren nach einem der vorhergehenden Ansprüche, **dadurch gekennzeichnet, dass** der Fermentationsansatz mit Vitamin D3 angereichert wird.

7. Verfahren nach einem der vorhergehenden Ansprüche, **dadurch gekennzeichnet, dass** der Fermentationsansatz mit Colostrum angereichert wird.

8. Verfahren nach einem der vorhergehenden Ansprüche, **dadurch gekennzeichnet, dass** der Fermentationsansatz mit Ölsäure angereichert wird.

9. Verfahren nach einem der vorhergehenden Ansprüche, **dadurch gekennzeichnet, dass** die Fermentation unter einer Begasung erfolgt.

10. Verfahren nach einem der vorhergehenden Ansprüche, **dadurch gekennzeichnet, dass** die Fermentation unter Rühren erfolgt.

11. Verfahren nach Anspruch 10, **dadurch gekennzeichnet, dass** die Fermentation unter ständigem Rühren erfolgt.

12. Verfahren nach einem der vorhergehenden Ansprüche, **dadurch gekennzeichnet, dass** die trockene Darreichungsform einer GcMAF-enthaltenden Formulierung beschichtet wird.

## Claims

1. Method for producing dry dosage forms of a GcMAF-containing formulation, **characterised in that**
- starter cultures are introduced into milk such that a fermentation preparation is produced, wherein fermentation then occurs in the milk,
- the fermentation product is heated prior to convective drying and
- the fermentation product is subsequently convectively dried.

2. Method according to claim 1, **characterised in that** the fermentation preparation stands for two days.

3. Method according to claim 2, **characterised in that** the fermentation preparation is stored at a temperature of 37 °C for two days.

4. Method according to any one of the preceding claims, **characterised in that** the fermentation product is heated to a temperature of 35 °C prior to convective drying.

5. Method according to any one of the preceding claims, **characterised in that** the fermentation product is sprayed onto carrier material.

6. Method according to any one of the preceding claims, **characterised in that** the fermentation preparation is enriched with vitamin D3.

7. Method according to any one of the preceding claims, **characterised in that** the fermentation preparation is enriched with colostrum.

8. Method according to any one of the preceding claims, **characterised in that** the fermentation preparation is enriched with oleic acid.

9. Method according to any one of the preceding claims, **characterised in that** the fermentation takes place under fumigation.

10. Method according to any one of the preceding claims, **characterised in that** the fermentation takes place while stirring.

11. Method according to claim 10, **characterised in that** the fermentation takes place while stirring constantly.

12. Method according to any one of the preceding claims, **characterised in that** the dry dosage form is coated with a GcMAF-containing formulation.

## Revendications

1. Procédé servant à fabriquer des formes galéniques sèches d'une formulation contenant du GcMAF, **caractérisé en ce**
- **que** des cultures de démarrage sont ajoutées dans du lait de manière à obtenir une préparation de fermentation, dans lequel une fermentation dans le lait est effectuée par la suite,
- le produit de fermentation est chauffé avant le séchage par convection, et
- **qu'**ensuite le produit de fermentation est séché par convection.

2. Procédé selon la revendication 1, **caractérisé en ce que** la préparation de fermentation repose pendant deux jours.

3. Procédé selon la revendication 2, **caractérisé en ce que** la préparation de fermentation est stockée pendant deux jours à une température de 37 °C.

4. Procédé selon l'une quelconque des revendications précédentes, **caractérisé en ce que** le produit de fermentation est chauffé avant le séchage par convection à une température de 35 °C.

5. Procédé selon l'une quelconque des revendications précédentes, **caractérisé en ce que** le produit de fermentation est aspergé sur un matériau de support.

6. Procédé selon l'une quelconque des revendications précédentes, **caractérisé en ce que** la préparation de fermentation est enrichie en vitamine D3.

7. Procédé selon l'une quelconque des revendications précédentes, **caractérisé en ce que** la préparation de fermentation est enrichie en colostrum.

8. Procédé selon l'une quelconque des revendications précédentes, **caractérisé en ce que** la préparation de fermentation est enrichie en acide oléique.

9. Procédé selon l'une quelconque des revendications précédentes, **caractérisé en ce que** la fermentation est effectuée sous un gazage.

10. Procédé selon l'une quelconque des revendications précédentes, **caractérisé en ce que** la fermentation est effectuée par agitation.

11. Procédé selon la revendication 10, **caractérisé en ce que** la fermentation est effectuée par agitation permanente.

12. Procédé selon l'une quelconque des revendications précédentes, **caractérisé en ce que** la forme galénique sèche d'une formulation contenant du GcMAF est enrobée.
